# FASCICULE DE BREVET EUROPEEN

(11) **EP 1 032 414 B1**
(45) Date de publication et mention de la délivrance du brevet: **07.05.2003**
(21) Numéro de dépôt: 98913813.6
(22) Date de dépôt: 03.03.1998
(51) Int. Cl.: A61K 38/55, A61K 31/405

(54) **COMBINAISON D'UN INHIBITEUR DE L'ENZYME DE CONVERSION DE L'ANGIOTENSINE ET D'UN DIURETIQUE POUR LE TRAITEMENT DES DESORDRES MICROCIRCULATOIRES**
KOMBINATION VON ANGIOTENSING-CONVERTIN-ENZYME HEMMERN MIT DIURETIKUM ZUR BEHANDLUNG VON STÖRUNGEN DER MIKROZIRKULATION
COMBINATION OF HYPERTENSIN CONVERTING ENZYME INHIBITOR WITH A DIURETIC FOR TREATING MICROCIRCULATION DISORDERS

(30) Priorité: 19.11.1997 FR 9714485
(43) Date de publication de la demande: 06.09.2000
(73) Titulaire: LES LABORATOIRES SERVIER, 92200 Neuilly sur Seine (FR)
(72) Inventeur: GUEZ, David, F-92200 Neuilly sur Seine (FR); SCHIAVI, Pierre, F-92000 Nanterre (FR); LEVY, Bernard, F-75010 Paris (FR)
(86) Numéro de dépôt international: FR9800411
(87) Numéro de publication internationale: WO99025374

(56) Documents cités:
- WO-A-93/17685
- WO-A-96/28185
- DATABASE BIOSIS BIOSCIENCES INFORMATION SERVICE, PHILADELPHIA, PA, US LUCCIONI R ET AL: "An equivalence study of the safety and efficacy of a fixed-dose combination of perindopril with indapamide versus fixed-dose combinations of captopril with hydrochlorothiazide and enalapril with hydrochlorothiazide in the treatment of hypertension" XP002071800 & JOURNAL OF HYPERTENSION, 13 (12 PART 2). 1995. 1847-1851.,
- DATABASE BIOSIS BIOSCIENCES INFORMATION SERVICE, PHILADELPHIA, PA, US CHALMERS J ET AL: "Blood pressure lowering for the secondary prevention of stroke: Rationale and design for PROGRESS" XP002071801 & JOURNAL OF HYPERTENSION, 14 (SUPPL. 2). 1996. S41-S46.,
- DATABASE BIOSIS BIOSCIENCES INFORMATION SERVICE, PHILADELPHIA, PA, US CHALMERS J ET AL: "Progress-perindopril protection against recurrent stroke study: Status in July 1996" XP002071802 & JOURNAL OF HYPERTENSION, 14 (SUPPL. 6). 1996. S47-S51.,
- DATABASE BIOSIS BIOSCIENCES INFORMATION SERVICE, PHILADELPHIA, PA, US NEAL B ET AL: "Blood pressure lowering in patients with cerebrovascular disease: Results of the PROGRESS (perindopril protection against recurrent stroke study) pilot phase" XP002071803 & CLINICAL AND EXPERIMENTAL PHARMACOLOGY AND PHYSIOLOGY, 23 (5). 1996. 444-446.,
- DATABASE MEDLINE US NATIONAL LIBRARY OF MEDICINE (NLM), BETHESDA, MD, US WHITLOCK G ET AL: "Blood pressure lowering for the prevention of cognitive decline in patients with cerebrovascular disease. PROGRESS Management Committee. Perindopril Protection Against Recurrent Stroke Study." XP002071804 & CLIN EXP HYPERTENS, JUL-AUG 1997, 19 (5-6) P843-55, UNITED STATES,
- DATABASE MEDLINE US NATIONAL LIBRARY OF MEDICINE (NLM), BETHESDA, MD, US NEAL B ET AL: "PROGRESS (perindopril protection against recurrent stroke study): rationale and design. PROGRESS Management Committee [corrected] [published erratum appears in J Hypertens 1996 Apr;14(4):535]" XP002071805 & J HYPERTENS, DEC 1995, 13 (12 PT 2) P1869-73, ENGLAND,

## Description

La présente invention concerne l'utilisation d'une combinaison d'un inhibiteur de l'enzyme de conversion de l'angiotensine (IEC) et d'un diurétique pour l'obtention de compositions pharmaceutiques destinées au traitement des désordres microcirculatoires artériolo-capillaires.

On sait que la plupart des maladies vasculaires dégénératives, l'hypertension artérielle par exemple (N.M. KAPLAN, "*Microvascular Rarefaction*", Clinical Hypertension 6^{th} ed. Baltimore Wilkinson et Wilkins, 1994, 86 ; A.S. GREENE et coll., "*Microvascular rarefaction and tissue vascular resistance in hypertension",* Am J. Physiol., 1989, 256 (Heart Circ. Physiol., 25):H 126-H 31 ; A.S. IZZARD et coll., *"Hypertension and the vasculature : arterioles and the myogenic response",* J. Hypertens., 1995, 13:1-4 ; A.M. HEAGERTY et coll., "*Small artery structure in hypertension*", Hypertension, 1993, 21:391-7), mais aussi les complications vasculaires de certaines maladies métaboliques, le diabète sucré par exemple (G. REACH et coll., "*Causes et mécanismes de la microanglopathie et de la neuropathie- "L'hypothèse glucose" et ses implications*", In : G. Tchobroutsky, G. Slama, R. Assan, P. Freychet, Paris : Pradel, 1990, 448-57), ou certaines dyslipidémies (J.F. TOOLE, "*Atherosclerosis*", Cerebrovascular disorders, New York : Raven Press, 1984, 199-213), s'accompagnent d'altérations anatomiques et/ou foncitonnelles de la microcirculation artériolo-capillaire (J.C.M.L. LE NOBLE et coll., "A functional morphometric study of the cremaster muscle microcirculation in young spontaneously hypertensive rats", J. Hypertens., 1990, 8:741-8 ; I.I.H. CHEN et coll., "*Microvascular rarefaction in spontaneously hypertensive rat cremaster muscle",* Am. J. Physiol, 1981, 241:H 306-10).

Les altérations anatomiques et/ou fonctionnelles de la microcirculation artériolo-capillaire peuvent prendre différents aspects, comme par exemple :
- une raréfaction artériolo-capillaire (P. GASSER, "*Nailfold microcirculation in normotensive and essential hypertensive subjects as assessed by video-microscopy*", J. Hypertens., 1992, 1:83-6),
- un défaut de recrutement artériolo-capillaire (B.W. ZWEIFACH, "*Micropressure-flow relationships in a skeletal muscle of spontaneously hypertensive rats*", Hypertension, 1981, 3:601-14),
- et de façon plus générale, une inadaptation de la distribution du sang dans les tissus en fonction des besoins métaboliques, toute altération susceptible d'induire ou de péréniser une hypoperfusion tissulaire, une ischémie, absolue ou relative (E. VICAUT, "*Hypertension and the microcirculation : a brief overview of experimental studies*", J. Hypertens., 1992, 10 suppl 5:S59-S68).

On sait également que les altérations anatomiques et/ou fonctionnelles de la microcirculation artériolo-capillaire sus décrites peuvent précéder, par exemple, l'élévation des chiffres tensionnels dans l'hypertension artérielle, créant pour certains un véritable cercle vicieux (A.J. ZWEIFLER et coll., *"Diminished finger pulse in borderline hypertension : évidence for early structural vascular abnormality"*, Am. Heart J., 1982, 104-812-15 ; J.M. SULLIVAN et coll., *"Attenuation of the microcirculation in young pateints with high - output borderline hypertension",* Hypertension, 1983, 5:844-51).

On sait enfin que de très nombreux facteurs sont impliqués à la fois dans la régulation de l'hémodynamique générale (débits, résistances, pressions...) et dans la régulation, ou plutôt l'adaptation, de la distribution du sang dans les tissus en fonction du contexte (hiérarchisation dépendant de la nature des organes...) et des besoins métaboliques du moment (M.J. MULVANY, "The structure of the resistance vasculature in essential hypertension", J. Hypertens., 1987, 5:129:H ; H.A.J. STRUIJKER BOUDIER et coll., *"The microcirculation and hypertension*", J. Hypertens., 1992, 10 (suppl. 7) :S147-S156).

De très nombreuses substances vaso-actives ont été identifiées, avec un intérêt tout particulier au cours de ces dernières années pour, à titre d'exemple, des substances d'origine ou à effet sur les fibres musculaires lisses et l'endothélium vasculaire (S. LAURENT et coll., "*Physiopathologie et pharmacologie du remodelage artériel dans l'hypertension artérielle*", La lettre du pharmacologue, 1997, 11:146-54 ; TADDEI et coll., *"Hypertension causes prematurate aging of endothelial function in humans*", Hypertension, 1997, 29:736-43).

La complexité de ces différentes régulations, le nombre de facteurs mis en jeu, leur interactivité aujourd'hui mieux comprise nous ont conduit à proposer la combinaison de plusieurs médicaments directement ou indirectement vaso-actifs pour à la fois prévenir et traiter :
- d'une part, l'atteinte clinique, l'hypertension artérielle par exemple, lorsque l'élévation des chiffres tensionnels atteint, voire dépasse, les normes recommandées par la Communauté Scientifique Internationale ;
- d'autre part, son retentissement sur la perfusion tissulaire dans le cadre de désordres macro/microcirculatoires qui, on le sait, peuvent précéder, entretenir et aggraver l'entité clinique précédemment décrite à titre d'exemple (l'hypertension artérielle, mais aussi, par exemple, les complications vasculaires de certaines maladies métaboliques...) (H.A.J. STRUUKER-BOUDIER et coll., "*Assessment of the microcirculation in cardiovascular disease*", Clin. Sci., 1996, 91:131-9).

L'action de différentes substances vaso-actives peut donc se compléter utilement, et fournir un effet thérapeutique amélioré pour le traitement de fond des maladies vasculaires dégénératives, voire pour la prévention des incidents et accidents qu'elles induisent.

On sait aussi que certains IEC ont un effet bénéfique sur la microcirculation artériolaire ou coronarienne, mais à aucun moment il n'a été démontré dans la littérature d'effet bénéfique sur l'unité fonctionnelle que représente une artériole et les capillaires adjacents.

Or, il a été montré, ce qui est l'objet de la présente invention, que la combinaison d'un IEC avec un diurétique, en dehors des propriétés connues de cette association, permettait de façon surprenante de corriger les désordres microcirculatoires à la fois au niveau artériolaire et capillaire alors qu'aucune propriété de cet ordre n'a jamais été ni décrite ni revendiquée dans les publications ou brevets antérieurs relatifs à des combinaisons, en particulier d'IEC et de diurétiques, aux IEC ou aux diurétiques.

Ce type de combinaison d'agents vaso-actifs trouve en plus son originalité dans le fait, en particulier, que chacun des constituants de la combinaison est le plus souvent utilisé à des doses faibles, généralement inférieures à celles utilisées dans chacun de leurs indications princeps.

Ce type d'association trouve donc son utilité dans l'obtention de compositions pharmaceutiques utiles pour le traitement des désordres microcirculatoires artériolo-capillaires. Ces compositions peuvent donc être utilisées dans toutes les pathologies où des désordres microcirculatoires interviennent comme par exemple les maladies vasculaires dégénératives, l'hypertension artérielle, l'insuffisance cardiaque, l'ischémie cérébrale, les accidents coronariens, l'artérite des membres inférieurs, la prévention et le traitement des complications cardiovasculaires du diabète de type II, les rétinopathies, les néphropathies, etc... et cela en traitement principal ou secondaire.

Les IEC utilisables dans ces compositions sont, à titre non limitatif : le Perindopril, le Captopril, l'Enalapril, le Lisinopril, le Delapril, le Fosinopril, le Quinapril, le Ramipril, le Spirapril, l'Imidapril, le Trandolapril, le Benazepril, le Cilazapril et le Temocapril, et leurs sels d'addition à un acide ou à une base pharmaceutiquement acceptable.

Les IEC préférés sont le Perindopril, le Captopril, l'Enalapril, le Lisinopril, le Benazapril, le Quinapril et le Delapril et leurs sels et plus spécialement le Perindopril et ses sels.

Les diurétiques utilisables dans ces compositions sont, à titre non limitatif : l'Indapamide, l'Hydrochlorothiazide, le Furosemide, l'Altizide, le Trichlorméthiazide, le Triflumethazide, le Bemetizide, le Cyclothiazide, le Methylclothiazide, l'Azosemide, le Chlorothiazide, le Butizide, le Bendrofluazide, le Cyclopenthiazide, le Benzchlortriazide, le Polythiazide, l'Hydroflumethazide, le Benzthiazide, l'Ethiazide, le Penflutazide, la Clopamide, la Ciclétamide ou le Pirétanide, et leurs sels d'addition à un acide ou à une base pharmaceutiquement acceptable.

Les diurétiques préférés sont l'Indapamide et l'Hydrochlorothiazide et leurs sels et plus spécialement l'Indapamide et ses sels.

L'invention concerne donc plus préférentiellement l'utilisation d'une combinaison de l'inhibiteur d'enzyme de conversion Perindopril ou de l'un de ses sels d'addition à une base pharmaceutiquement acceptable et du diurétique Indapamide pour l'obtention de compositions pharmaceutiques destinées au traitement des désordres microcirculatoires artériolo-capillaires.

Les compositions pharmaceutiques seront présentées sous des formes pharmaceutiques convenant pour l'administration par voie orale, parentérale et notamment intraveineuse, per ou transcutanée, nasale, rectale, perlinguale, oculaire, respiratoire et plus spécifiquement les comprimés, les comprimés sublinguaux, les gélules, les glossettes, les capsules, les tablettes, les préparations injectables, les aérosols, les gouttes oculaires ou nasales, les suppositoires, les crèmes, pommades, gels dermiques, etc...

La voie d'administration préférée est la voie orale et les compositions pharmaceutiques correspondantes permettant la libération instantanée ou différée des principes actifs.

Les compositions pharmaceutiques préférées sont les comprimés.

Dans les compositions pharmaceutiques, les quantités d'IEC et de diurétique sont adaptées à la nature de ces principes actifs et leurs proportions relatives sont donc variables en fonction des principes actifs.

Lorsque l'IEC est le Perindopril sous forme de sel de tert-butylamine et que le diurétique est l'Indapamide, ces proportions sont respectivement comprises entre 65 et 85 % et entre 35 et 15 % de la masse totale des principes actifs et préférentiellement compris entre 70 et 80 % pour l'IEC et entre 30 et 20 % pour le diurétique.

Les pourcentages préférés pour cette combinaison sont 76 % de sel de tert-butylamine de Perindopril et 24 % d'Indapamide.

Les compositions, outre les principes actifs, contiennent un ou plusieurs véhicules ou excipients pharmaceutiquement acceptables.

Parmi les excipients pharmaceutiquement acceptables, on peut citer à titre non limitatif, les liants, les diluants, les délitants, les stabilisants, les conservateurs, les lubrifiants, les odorants, les aromatisants ou les édulcorants.

La posologie varie selon l'âge et le poids du patient, la voie d'administration, la nature de l'indication thérapeutique et des traitements associés. Elle s'échelonne entre 1 et 50 mg selon la nature de l'IEC et entre 0,5 et 25 mg selon la nature du diurétique en une ou plusieurs prises par 24 heures.

Exemples de compositions pharmaceutiques.

### EXEMPLE 1 : Comprimés de Perindopril / Indapamide

| *Constituants* | *Quantité (mg)* |
|---|---|
| Perindopril, sel de tert-butylamine | 2 |
| Indapamide | 0,625 |
| Silice colloïdale hydrophobe | 0,25 |
| Lactose | 64,175 |
| Stéarate de magnésium | 0,45 |
| Cellulose microcristalline | 22,5 |
| Pour un comprimé terminé à | 90 |

### EXEMPLE 2 :

| *Constituants* | *Quantité (mg)* |
|---|---|
| Perindopril, sel de tert-butylamine | 4 |
| Indapamide | 1,25 |
| Silice colloïdale hydrophobe | 0,25 |
| Lactose | 61,55 |
| Stéarate de magnésium | 0,45 |
| Cellulose microcristalline | 22,5 |
| Pour un comprimé terminé à | 90 |

### EXEMPLE 3 :

| *Constituants* | *Quantité (mg)* |
|---|---|
| Captopril | 50 |
| Hydrochlorothiazide | 25 |

### EXEMPLE 4 :

| *Constituants* | *Quantité (mg)* |
|---|---|
| Enalapril, maléate | 20 |
| Hydrochlorothiazide | 12,5 |

### EXEMPLE 5:

| *Constituants* | *Quantité (mg)* |
|---|---|
| Lisinopril | 20 |
| Hydrochlorothiazide | 12,5 |

### EXEMPLE 6 :

| *Constituants* | *Quantité (mg)* |
|---|---|
| Benazepril, chlorhydrate | 10 |
| Hydrochlorothiazide | 12,5 |

### EXEMPLE 7 :

| *Constituants* | *Quantité (mg)* |
|---|---|
| Quinapril, chlorhydrate | 20 |
| Hydrochlorothiazide | 12,5 |

### EXEMPLE 8 :

| *Constituants* | *Quantité (mg)* |
|---|---|
| Perindopril, sel de tert-butylamine | 2 |
| Hydrochlorothiazide | 12,5 |

### EXEMPLE 9 :

| *Constituants* | *Quantité (mg)* |
|---|---|
| Perindopril, sel de tert-butylamine | 4 |
| Hydrochlorothiazide | 25 |

### EXEMPLE 10 :

| *Constituants* | *Quantité (mg)* |
|---|---|
| Captopril | 50 |
| Indapamide | 1,25 |

### EXEMPLE 11 :

| *Constituants* | *Quantité (mg)* |
|---|---|
| Enalapril, maléate | 20 |
| Indapamide | 1,25 |

### EXEMPLE 12 :

| *Constituants* | *Quantité (mg)* |
|---|---|
| Lisinopril | 20 |
| Indapamide | 1,25 |

### EXEMPLE 13 :

| *Constituants* | *Quantité (mg)* |
|---|---|
| Benazépril, chlorhydrate | 10 |
| Indapamide | 1,25 |

### EXEMPLE 14 :

| *Constituants* | *Quantité (mg)* |
|---|---|
| Quinapril, chlorhydrate | 20 |
| Indapamide | 1,25 |

### EXEMPLE 15 :

| *Constituants* | *Quantité (mg)* |
|---|---|
| Perindopril, sel de tert-butylamine | 4 |
| Indapamide | 5 |

### EXEMPLE 16 :

| *Constituants* | *Quantité (mg)* |
|---|---|
| Captopril | 50 |
| Bendrofluazide | 5 |

### EXEMPLE 17 :

| *Constituants* | *Quantité (mg)* |
|---|---|
| Delapril, chlorhydrate | 30 |
| Indapamide | 2,5 |

### EXEMPLE 18 :

| *Constituants* | *Quantité (mg)* |
|---|---|
| Delapril, chlorhydrate | 30 |
| Hydrochlorothiazide | 25 |

### EXEMPLE 19 :

| *Constituants* | *Quantité (mg)* |
|---|---|
| Fosinopril | 10 |
| Hydrochlorothiazide | 25 |

### Etude pharmacologique des compositions selon l'invention

### Effets de la combinaison du sel de tert-butylamine de Perindopril (0,76 mg/kg/j, PO) + Indapamide (0,24 mg/kg/j, PO), chez le rat hypertendu réno-vasculaire 1R-1C : Aspect hémodynamique et études de la densité artériolo-capillaire sous endocardique.

Des rats Wistar âgés de 8 semaines (n = 56 ; poids corporel = 200 g) ont subi dans un premier temps la pose d'un clip (diamètre 0,2 mm) sur l'artère rénale gauche et quatre jours plus tard, une néphrectomie controlatérale était effectuée. Une série de rats identiques (n = 13) a subi les mêmes interventions (anesthésie + chirurgie) mais sans sténose de l'artère rénale ni néphrectomie (groupe contrôle NT). Les rats Goldblatt 1R-1C ont reçu :
- soit une alimentation normale : groupe contrôle HT ;
- soit une alimentation contenant du sel de tert-butylamine de perindopril (0,76 mg/kg/jour) et de l'indapamide (0,24 mg/kg/jour) : groupe HT + combinaison.

Les effectifs de chaque groupe ont été ajustés, en tenant compte de la mortalité spécifique dans chaque groupe de manière à pouvoir analyser, après 4 semaines de traitement, au moins 9 animaux par groupe.

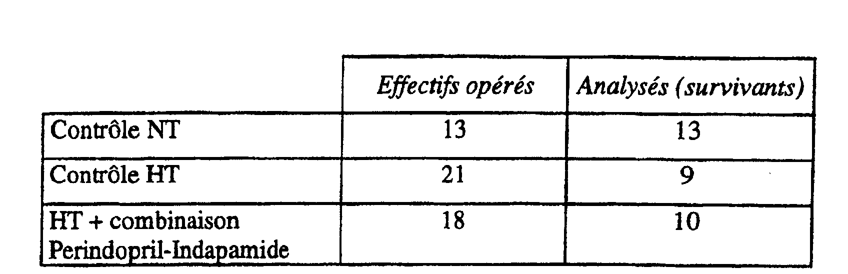

Après 4 semaines de traitement, on enregistrait sous anesthésie les paramètres hémodynamiques (Table I), puis on prélevait le coeur pour analyse histomorphométrique quantitative.
La combinaison sel de Perindopril-Indapamide diminuait significativement la pression artérielle (p<0,01). Les débit et fréquence cardiaques n'étaient pas modifiés par le traitement.
Le degré d'hypertrophie ventriculaire gauche (poids du VG/poids corporel) était significativement diminué par rapport au groupe contrôle HT (p<0,001).

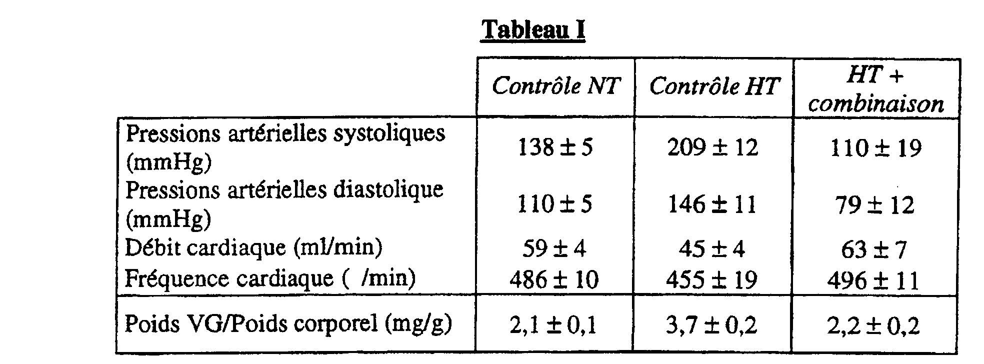

### Densité capillaire sous endocardique dans la paroi du ventricule gauche

La densité capillaire était significativement diminuée dans le groupe contrôle HT par rapport au groupe contrôle NT (p<0,05) et normalisée par la combinaison sel de Perindopril-Indapamide.

### Densité artériollaire sous endocardique dans la paroi du ventricule gauche

Le nombre d'artérioles par mm² de surface sous endocardique était significativement augmenté dans le groupe contrôle HT (p<0,05) et normalisé dans le groupe combinaison sel de Perindopril-Indapamide.

Les résultats sont présentés dans le tableau II.

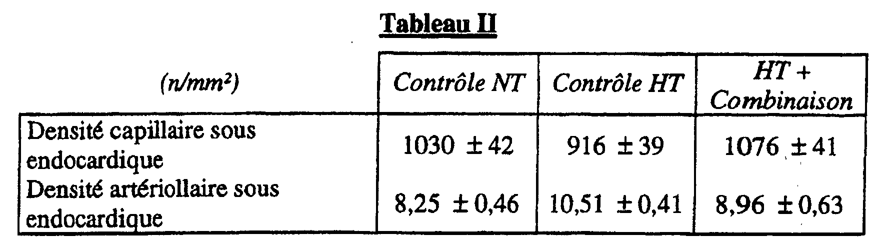

On peut donc interpréter les données qui précèdent de la façon suivante :
- Il se confirme qu'il existe dans la plupart des maladies vasculaires dégénératives, ici dans l'hypertension artérielle, des altérations anatomiques et/ou fonctionnelles de la microcirculation artériolo-capillaire.
- Dans l'expérience ici réalisée, l'anomalie la plus nette concerne la densité capillaire fortement diminuée chez les hypertendus, et normalisée sous l'effet du "traitement" par la combinaison de ces deux principes actifs.
- La part respective de l'effet de chacun des constituants de l'association sur les artérioles et les capillaires est, compte tenu des conditions de réalisation de l'expérience, difficile à définir. Il semble cependant que chacun des constituants ait un rôle à la fois sur la composante artériolaire et la composante capillaire de l'unité fonctionnelle microcirculatoire.

En conclusion, le fait de combiner le sel de Perindopril et l'Indapamide, dans les proportions 76-24 %, normalise les densités capillaire et artéiolaire sous endocardiques, étudiées ici pour illustrer l'invention.

## Revendications

1. Utilisation d'une combinaison d'un inhibiteur de l'enzyme de conversion de l'angiotensine et d'un diurétique pour l'obtention de compositions pharmaceutiques destinées au traitement des désordres microcirculatoires artériolo-capillaires.

2. Utilisation selon la revendication 1 **caractérisée en ce que** l'inhibiteur d'enzyme de conversion est le Perindopril, le Captopril, l'Enalapril, le Lisinopril, le Delapril, le Fosinopril, le Quinapril, le Ramipril, le Spirapril, l'Imidapril, le Trandolapril, le Benazepril, le Cilazapril ou le Temocapril et que le diurétique est l'Indapamide, l'Hydrochlorothiazide, le Furosemide, l'Altizide, le Trichlorméthiazide, le Triflumethazide, le Bemetizide, le Cyclothiazide, le Methylclothiazide, l'Azosemide, le Chlorothiazide, le Butizide, le Bendrofluazide, le Cyclopenthiazide, le Benzchlortriazide, le Polythiazide, l'Hydroflumethazide, le Benzthiazide, l'Ethiazide, le Penflutazide, la Clopamide, la Ciclétamide ou le Pirétanide,
ainsi que les sels d'addition de ces composés à un acide ou une base pharmaceutiquement acceptable.

3. Utilisation selon la revendication 2 **caractérisée en ce que** l'inhibiteur d'enzyme de conversion est le Perindopril, le Captopril, l'Enalapril, le Lisinopril, le Benazepril, le Quinapril ou le Delapril et que le diurétique est l'Indapamide ou l'Hydrochlorothiazide ainsi que les sels d'addition de ces composés à un acide ou à une base pharmaceutiquement acceptable.

4. Utilisation d'une combinaison de l'inhibiteur d'enzyme de conversion Perindopril ou de l'un de ses sels d'addition à une base pharmaceutiquement acceptable et du diurétique Indapamide pour l'obtention de compositions pharmaceutiques destinées au traitement des désordres microcirculatoires artériolo-capillaires.

5. Utilisation selon la revendication 4 caractérisée cn ce que l'inhibiteur d'enzyme de conversion est le sel de tert-butylamine de Perindopril et que le diurétique est l'Indapamide.

6. Utilisation selon la revendication 4 **caractérisée en ce que** les compositions pharmaceutiques renferment des quantités de sel de tert-butylamine de Perindopril et d'Indapamide respectivement comprises entre 65 et 85 % et entre 35 et 15 % de la masse totale des principes actifs.

7. Utilisation selon la revendication 6 **caractérisée en ce que** ces quantités sont 76 % de sel de tert-butylamine de Perindopril et 24 % d'Indapamide.

8. Utilisation selon la revendication 1 **caractérisée en ce que** les compositions pharmaceutiques se présentent sous la forme de comprimés.

9. Utilisation selon la revendication 4 **caractérisée en ce que** les compositions pharmaceutiques se présentent sous la forme de comprimés.

## Claims

1. Use of a combination of an angiotensin-converting enzyme inhibitor and of a diuretic in the preparation of pharmaceutical compositions for the treatment of arteriolo-capillary microcirculatory disorders.

2. Use according to claim 1, **characterised in that** the converting enzyme inhibitor is perindopril, captopril, enalapril, lisinopril, delapril, fosinopril, quinapril, ramipril, spirapril, imidapril, trandolapril, benazepril, cilazapril or temocapril and **in that** the diuretic is indapamide, hydrochlorothiazide, furosemide, altizide, trichlormethiazide, triflumethazide, bemetizide, cyclothiazide, methylclothiazide, azosemide, chlorothiazide, butizide, bendrofluazide, cyclopenthiazide, benzchlortriazide, polythiazide, hydroflumethazide, benzthiazide, ethiazide, penflutazide, clopamide, cicletamide or piretanide,
or an addition salt of such a compound with a pharmaceutically acceptable acid or base.

3. Use according to claim 2, **characterised in that** the converting enzyme inhibitor is perindopril, captopril, enalapril, lisinopril, benazepril, quinapril or delapril and **in that** the diuretic is indapamide or hydrochlorothiazide, or an addition salt of such a compound with a pharmaceutically acceptable acid or base.

4. Use of a combination of the converting enzyme inhibitor perindopril, or an addition salt thereof with a pharmaceutically acceptable base, and of the diuretic indapamide in the preparation of pharmaceutical compositions for the treatment of arteriolo-capillary microcirculatory disorders.

5. Use according to claim 4, **characterised in that** the converting enzyme inhibitor is the tert-butylamine salt of perindopril and **in that** the diuretic is indapamide.

6. Use according to claim 4, **characterised in that** the pharmaceutical compositions comprise the tert-butylamine salt of perindopril and indapamide in amounts of from 65 to 85 % and from 35 to 15 % of the total weight of the active ingredients, respectively.

7. Use according to claim 6, **characterised in that** the amounts are 76 % of the tert-butylamine salt of perindopril and 24 % of indapamide.

8. Use according to claim 1, **characterised in that** the pharmaceutical compositions are in the form of tablets.

9. Use according to claim 4, **characterised in that** the pharmaceutical compositions are in the form of tablets.

## Patentansprüche

1. Verwendung einer Kombination aus einem Inhibitor des Angiotensin umwandelnden Enzyms und einem Diuretikum für die Herstellung von pharmazeutischen Zubereitungen zur Behandlung von Störungen der arteriolo-kapillaren Mikrozirkulation.

2. Verwendung nach Anspruch 1, **dadurch gekennzeichnet, daß** der Inhibitor für das Umwandlungsenzym Perindopril, Captopril, Enalapril, Lisinopril, Delapril, Fosinopril, Chinapril, Ramipril, Spiripril, Imidapril, Trandolapril, Benazepril, Cilazapril oder Temocapril und das Diuretikum Indapamid, Hydrochlorthiazid. Furosemid, Altizid, Trichlormethiazid, Triflumethazid, Bemetizid, Cyclothiazid, Methylcyclothiazid, Azosemid, Chlorthlazid, Butizid, Bendrofluazid, Cyclopenthiazid, Benzchlortriazid, Polythiazid, Hydroflumethiazid, Benzthiazid, Ethiazid, Penflutazid, Clopamid, Cicletamid oder Piretanid ist,
oder ein Additionssalz dieser Verbindungen mit einer pharmazeutisch annehmbaren Säure oder Base.

3. Verwendung nach Anspruch 2, **dadurch gekennzeichnet, daß** der Inhibitor des Umwandlungsenzyms Perindopril, Captopril, Enalapril, Lisinopril, Benazepril, Chinapril oder Delapril und das Diuretikum Indapamid oder Hydrochlorthiazid oder die Additionssalze dieser Verbindungen mit einer pharmazeutisch annehmbaren Säure oder Base sind.

4. Verwendung einer Kombination aus dem Inhibitor des Umwandlungsenzyms Perindopril oder eines seiner Additionssalze mit einer pharmazeutisch annehmbaren Säure und des Diuretikums Indapamid für die Herstellung von pharmazeutischen Zubereitungen zur Behandlung von Störungen der arteriolo-kapillaren Mikrozirkulation.

5. Verwendung nach Anspruch 4, **dadurch gekennzeichnet, daß** der Inhibitor der Umwandlungsenzyms das tert.-Butylaminsalz von Perindopril und das Diuretikum Indapamid sind.

6. Verwendung nach Anspruch 4, **dadurch gekennzeichnet, daß** die pharmazeutischen Zubereitungen das tert.-Butylaminsalz von Perindopril beziehungsweise Indapamid in Mengen zwischen 65 und 85 % bzw. zwischen 35 und 15 %, bezogen auf die Gesamtmasse der Wirkstoffe, enthalten.

7. Verwendung nach Anspruch 6, **dadurch gekennzeichnet, daß** die Mengen 76% für das tert.-Butylaminsalz von Perindopril und 24 % für Indapamid betragen.

8. Verwendung nach Anspruch 1, **dadurch gekennzeichnet, daß** die pharmazeutischen Zubereitungen in Form von Tabletten vorliegen.

9. Verwendung nach Anspruch 4, **dadurch gekennzeichnet, daß** die pharmazeutischen Zubereitungen in Form von Tabletten vorliegen.
